# EUROPÄISCHE PATENTSCHRIFT

(11) **EP 0 882 049 B1**
(45) Veröffentlichungstag und Bekanntmachung des Hinweises auf die Patenterteilung: **20.11.2002**
(21) Anmeldenummer: 97903260.4
(22) Anmeldetag: 12.02.1997
(51) Int. Cl.: C07D 471/04, A61K 31/44

(54) **GEGEBENENFALLS SUBSTITUIERTE 8-CYAN-1-CYCLOPROPYL-7-(2,8-DIAZABICYCLO- 4.3.0]-NONAN-8-YL)-6-FLUOR-1,4-DIHYDRO-4-OXO-3-CHINOLINCARBONSÄUREN UND IHRE DERIVATE**
POSSIBLY SUBSTITUTED 8-CYANO-1-CYCLOPROPYL-7-(2,8-DIAZABICYCLO- 4.3.0]-NONAN-8-YL)-6-FLUORO-1,4-DIHYDRO-4-OXO-3-QUINOLIN CARBOXYLIC ACIDS AND THEIR DERIVATIVES
ACIDES 8-CYANO-1-CYCLOPROPYL-7-(2,8-DIAZABICYCLO-(4.3.0)-NONAN-8-YL)-6-FLUORO-1,4-DIHYDRO-4-OXO-3-QUINOLINOCARBOXYLIQUES EVENTUELLEMENT SUBSTITUES ET LEURS DERIVES

(30) Priorität: 23.02.1996 DE 19606762; 22.08.1996 DE 19633805
(43) Veröffentlichungstag der Anmeldung: 09.12.1998
(60) Teilanmeldung: 02006519.9
(73) Patentinhaber: BAYER AG, 51368 Leverkusen (DE)
(72) Erfinder: BARTEL, Stefan, D-51465 Bergisch Gladbach (DE); JAETSCH, Thomas, D-50668 Köln (DE); HIMMLER, Thomas, D-51519 Odenthal (DE); RAST, Hans-Georg, D-51465 Bergisch Gladbach (DE); HALLENBACH, Werner, D-40789 Monheim (DE); HEINEN, Ernst, D-51109 Köln (DE); PIRRO, Franz, D-40764 Langenfeld (DE); SCHEER, Martin, D-42113 Wuppertal (DE); STEGEMANN, Michael, Kansas City, MO 64112 (US); STUPP, Hans-Peter, D-51379 Leverkusen (DE); WETZSTEIN, Heinz-Georg, D-51377 Leverkusen (DE)
(86) Internationale Anmeldenummer: EP9700637
(87) Internationale Veröffentlichungsnummer: WO97031001

(56) Entgegenhaltungen:
- EP-A- 0 391 132

## Beschreibung

Die vorliegende Erfindung betrifft die neue gegebenenfalls substituierte 8-Cyan-1-cyclopropyl-7-(2,8-diazabicyclo[4.3.0]nonan-8-yl)-6-fluor-1,4-dihydro-4-oxo-3-chinolincarbonsäuren, sowie ihre Derivate, Verfahren zu ihrer Herstellung sowie diese enthaltende antibakterielle Mittel.

Chinolincarbonsäuren und ihre antibakterielle Wirkung sind bereits bekannt geworden. So sind Ofloxacin, Norfloxacin, Enrofloxacin und Danofloxacin Wirkstoffe aus dieser Stoffklasse, die breite Verwendung in der Tiermedizin finden. Ihr Anwendung ist jedoch nicht immer zufriedenstellend.

EP-A-0 391 132 offenbart Chinoloncarbonsäure-Derivate, die in 5-Position einen Alkylrest tragen. Für die Substituenten in 7- und 8-Position wird ein breites Spektrum an möglichen Bedeutungen angegeben. EP-A-0 276 700 offenbart 8-Cyano-1-cyclopropyl-chinoloncarbonsäure-Derivate, die in 7-Position verschiedene Aminsubstituenten tragen können.

Die vorliegende Erfindung betrifft gegebenenfalls substituierte 8-Cyan-1-cyclopropyl-7-(2,8-diazabicyclo[4.3.0]nonan-8-yl)-6-fluor-1,4-dihydro-4-oxo-3-chinolincarbonsäuren und ihre Derivate der allgemeinen Formel (I) 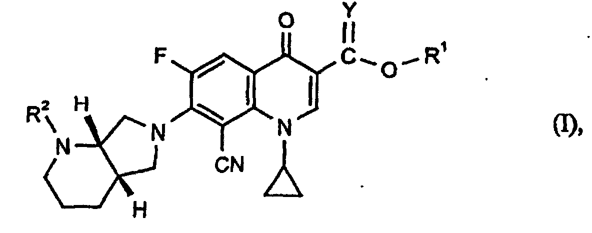 in welcher
- R¹: für Wasserstoff, gegebenenfalls durch Hydroxy, Methoxy, Amino, Methylamino oder Dimethylamino substituiertes C₁-C₄-Alkyl oder (5-Methyl-2-oxo-1,3-dioxol-4-yl)-methyl steht,
- R²: für Wasserstoff, Benzyl, C₁-C₃-Alkyl, (5-Methyl-2-oxo-1,3-dioxol-4-yl)methyl, Reste der Strukturen -CH=CH-COOR³, -CH₂CH₂COOR³, -CH₂CH₂CN, -CH₂CH₂COCH₃, -CH₂COCH₃, in denen R³ für Methyl oder Ethyl steht, oder einen Rest der allgemeinen Struktur R⁴-(NH-GHR⁵-CO)ₙ- steht, in der R⁴ für Wasserstoff, C₁-C₃-Alkyl oder den Rest -COO-tert.-Butyl steht, R⁵ für Wasserstoff, C₁-C₄-Alkyl, Hydroxyalkyl, Aminoalkyl, Thioalkyl, Carboxyalkyl oder Benzyl steht und n = 1 oder 2 ist,
und
- Y: für Sauerstoff steht.

Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- und Guanidiniumsalze vorliegen.

Die vorliegende Erfindung betrifft das Verfahren zur Herstellung von gegebenenfalls substituierten 8-Cyan-1-cyclopropyl-7-(2,8-diazabicyclo[4.3.0]nonan-8-yl)-6-fluor-1,4-dihydro-4-oxo-3-chinolincarbonsäuren, das dadurch gekennzeichnet ist, daß man Verbindungen der Formel (II) 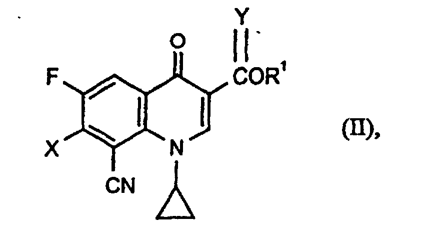 in welcher
- R¹ und Y: die oben angegebene Bedeutung hat und
- X: für Halogen, insbesondere fiir Fluor oder Chlor steht,
mit 2,8-Diazabicyclo[4.3.0]nonanen der Formel (III) 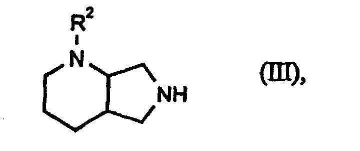 in welcher
- R²: die oben angegebene Bedeutung hat
gegebenenfalls in Gegenwart von Säurebindemitteln umsetzt. Gegebenenfalls wird anschließend der Carbonsäureester gespalten. Gegebenenfalls werden anschließend Verbindungen der Formel (I), in welcher R² für Wasserstoff steht, N-alkyliert, N-alkenyliert oder N-acyliert.

Die erfindungsgemäßen Verbindungen weisen im Vergleich zu bekannten Vertretern diesen Strukturtyps eine höhere antibakterielle Wirkung, insbesondere gegen E. coli, Staphylokokken, Streptokokken, Salmonellen und Mycoplasmen auf. Sie eignen sich daher als Wirkstoffe für die Human- und Veterinärmedizin. Vorteilhaft ist ihr rascher Abbau im Boden nach Ausscheidung durch den behandelten Organismus.

Bevorzugt sind Verbindungen der Formel (I),in welcher
- R¹: für Wasserstoff, gegebenenfalls durch Hydroxy, Methoxy, Amino, Methylamino oder Dimethylamino substituiertes C₁-C₄-Alkyl oder (5-Methyl-2-oxo-1,3-dioxol-4-yl)-methyl steht,
- R²: für Wasserstoff, Benzyl, C₁-C₃-Alkyl, (5-Methyl-2-oxo-1,3-dioxol-4-yl)methyl, Reste der Strukturen -CH=CH-COOR³, -CH₂CH₂COOR³, -CH₂CH₂CN, -CH₂COCH₃, in denen R³ für Methyl oder Ethyl steht, oder einen Rest der allgemeinen Struktur R⁴-(NH-CHR⁵-CO)ₙ- steht, in der R⁴ für Wasserstoff, C₁-C₃-Alkyl oder den Rest -COO-tert.-Butyl steht, R⁵ für Wasserstoff, C₁-C₄-Alkyl, Hydroxyalkyl, Aminoalkyl, Thioalkyl oder Benzyl steht und n = 1 oder 2 ist,
und
- Y: für Sauerstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind Verbindungen der Formel (I),in welcher
- R¹: für Wasserstoff, C₁-C₄-Alkyl oder (5-Methyl-2-oxo-1,3-dioxol-4-yl)-methyl steht,
- R²: für Wasserstoff, Methyl, (5-Methyl-2-oxo-1,3-dioxol-4-yl)-methyl, Reste der Strukturen -CH=CH-COOR³, -CH₂CH₂COOR³, -CH₂CH₂CN, -CH₂COCH₃, in denen R³ fiir Methyl oder Ethyl steht, oder einen Rest der allgemeinen Struktur R⁴-(NH-CHR⁵-CO)ₙ- steht, in der R⁴ für Wasserstoff, C₁-C₃-Alkyl oder den Rest -COO-tert.-Butyl steht, R⁵ für Wasserstoff, C₁-C₄-Alkyl, Hydroxyalkyl, Aminoalkyl, Thioalkyl oder Benzyl steht und n = 1 oder 2 ist,
und
- Y: für Sauerstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

Verwendet man zur Herstellung von Verbindungen der Formel (I) beispielsweise 7-Chlor-8-cyan-1-cyclopropyl-6-fluor-1,4-dihydro-4-oxo-3-chinolincarbonsäure und 2,8-Diazabicyclo[4.3.0]nonan, so kann der Reaktionsverlauf durch das folgende Formelschema wiedergegeben werden:

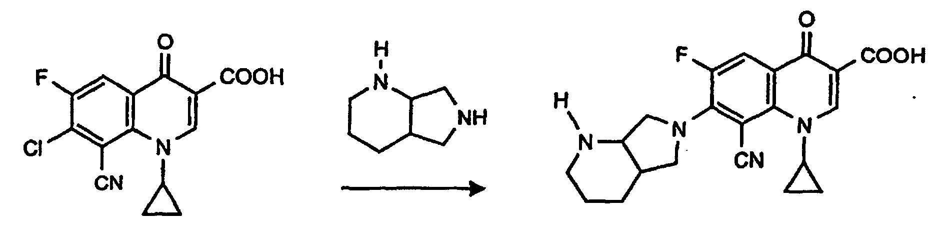

Verbindungen der Formel (I) können auch dadurch erhalten werden, daß man nach der Umsetzung einer Verbindung der Formel (II) mit 2,8-Diazabicyclo[4.3.0]nonan eine weitere Umsetzung des erhaltenen Produktes durchführt. So können Verbindungen der Formel (I) mit R² gleich einem Rest -CH=CH-COOEt beispielsweise nach folgendem Formelschema erhalten werden:

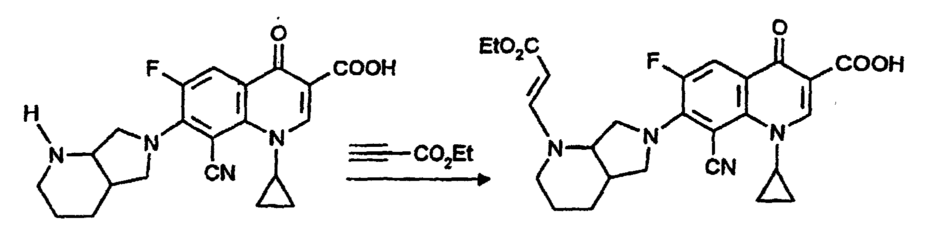

Verbindungen der Formel (I), in welcher R¹ für Wasserstoff steht, können in an sich bekannter Weise N-alkyliert, N-alkenyliert oder N-acyliert werden.

Zur N-Alkylierung werden die dem Rest R² entsprechenden Alkylhalogenide, -hydroxide oder die dem Rest R² entsprechenden Alkenyle verwendet.

Zur N-Alkenylierung werden die dem Rest R² entsprechenden Alkinyle verwendet.

Zur N-Acylierung werden die dem Rest R² entsprechenden Acylhalogenide, insbesondere Chloride, oder Anhydride, verwendet.

Als Alkylhalogenide seien genannt: Benzylchlorid, C₁₋₃-Alkyliodide, -bromide, -chloride, Chlorethancarbonsäuremethyl- oder -ethylester, Chloraceton;
als Alkenyle oder Alkinyle seien genannt: Propinylcarbonsäuremethyl- oder -ethylester, Acrylsäureethylester, Acrylsäurenitril;
als Acylhalogenide oder Anhydride seien genannt: Acetylchlorid, Pivaloylchlorid, N-tert.-Butyloxycarbonyl-L-alanin-N-carboxyanhydrid.

Die N-Alkylierung mit Alkylhalogeniden erfolgt vorzugsweise in einem Verdünnungsmittel wie z.B. Dimethylsulfoxid, N,N-Dimethylformamid, Sulfolan oder Acetonitril.

Als Säurebinder können übliche anorganische und organische Säurebindungsmittel wie beispielsweise Alkalihydroxide, Alkalicarbonate oder organische Amine verwendet werden.

Die Reaktionstemperaturen können dabei in einem größeren Bereich variiert werden. Im allgemeinen arbeitet man zwischen 20 und 200°C, vorzugsweise zwischen 50 und 150°C.

Die N-Alkylierung mit dem Rest R² entsprechenden Alkenylen und die N-Alkenylierung mit dem Rest R² entsprechenden Alkinylen erfolgt vorzugsweise in einem Verdünnungsmittel wie z.B. Dimethylsulfoxid, N,N-Dimethylformamid, N-Methyl-pyrrolidon, Glykol, Methylglykol oder Diethylenglykol.

Die Reaktionstemperaturen können dabei in einem größeren Bereich variiert werden. Im allgemeinen arbeitet mann zwischen 20 und 200°C, vorzugsweise zwischen 50 und 180°C.

Die N-Acylierung mit dem Rest R² entsprechenden Acylhalogeniden oder Anhydriden erfolgt vorzugsweise in einem Verdünnungsmittel wie z.B. Dimethylsulfoxid, N,N-Dimethylformamid, Sulfolan oder N-Methylpyrrolidon.

Die Umsetzung kann ohne ein Säurebindungsmittel oder auch in Gegenwart eines solchen durchgeführt werden.

Als Säurebinder können übliche anorganische und organische Säurebindungsmittel wie beispielsweise Triethylamin, 1,4-Diazabicyclo[2.2.2]octan und Diazabicyclo[5.4.0]undec-7-en eingesetzt werden.

Die Reaktionstemperaturen können dabei in einem größeren Bereich variiert werden. Im allgemeinen arbeitet man zwischen -10°C und 200°C, bevorzugt zwischen 0 und 150°C.

Die als Ausgangsverbindungen verwendeten Verbindungen der Formel (II) sind aus US-P 4 990 517 bekannt bzw. können nach bekannten Verfahren hergestellt werden. Z.B. seien genannt
7-Chlor-8-cyan-1-cyclopropyl-6-fluor-1,4-dihydro-4-oxo-3-chinolincarbonsäure
7-Chlor-8-cyan-1-cyclopropyl-6-fluor-1,4-dihydro-4-oxo-3-chinolincarbonsäuremethylester
8-Cyan-1-cyclopropyl-6,7-difluor-1,4-dihydro-4-oxo-3-chinolincarbonsäure
8-Cyan-1-cyclopropyl-6,7-difluor-1,4-dihydro-4-oxo-3-chinolincarbonsäureethylester.

So lassen sich die Verbindungen der Formel (II) z.B. herstellen, indem man eine Verbindung der Formel (IV) mit einem ß-Dimethylamino-acrylsäureester der Formel (V) umsetzt, das erhaltene Produkt der Formel (VI) mit Cyclopropylamin zu einer Verbindung der Formel (VII) umsetzt und anschließend die Verbindung (II) erhält:

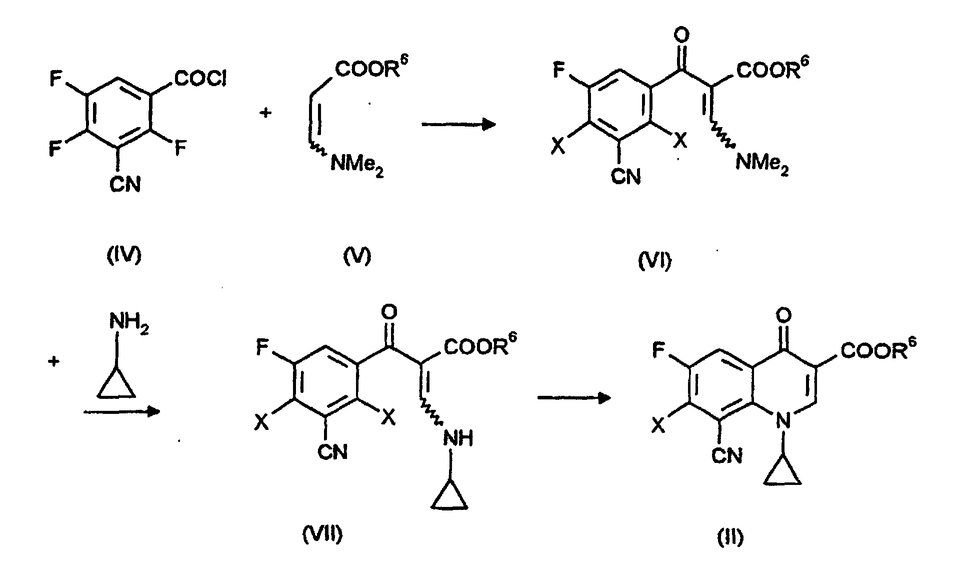

In obigen Formelschema steht
- X: für Halogen, insbesondere Fluor oder Chlor,
- R⁶: für C₁₋₄-Alkyl, insbesondere Methyl oder Ethyl, das gegebenenfalls wie bei R¹ angegeben, substituiert ist

Ebenso ist es möglich, eine Verbindung der Formel (IV) direkt mit β-Cyclopropylamino-acrylsäureester umzusetzen: 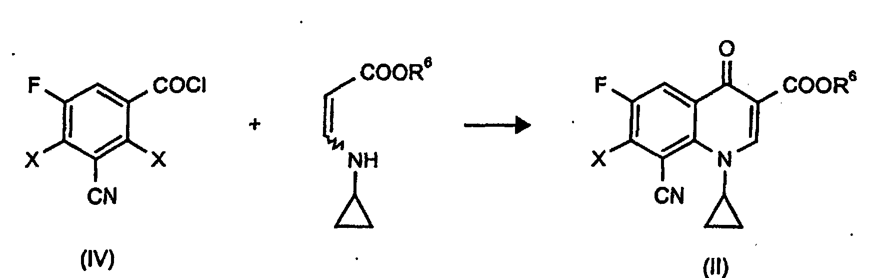 (In den Formeln haben X und R⁶ die oben angegebene Bedeutung.)

Dabei kann das z.B. Zwischenprodukt der Formel (IV) mit X = F nach folgendem Schema hergestellt werden:

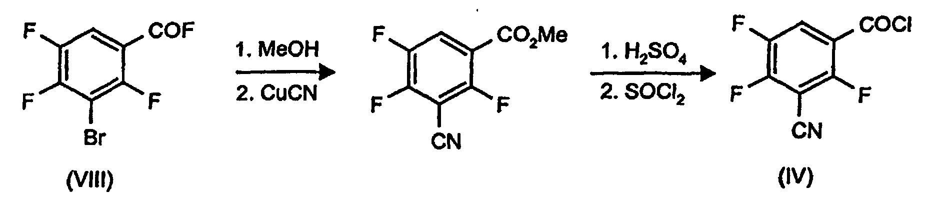

Die Verbindung der Formel VIII ist bekannt aus DE-A 3 631 906.

Die Umsetzung von Verbindungen der Formel (II) mit Verbindungen der Formel (III), bei der die Verbindungen (III) auch in Form ihrer Salze, wie z.B. der Hydrochloride, eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 0 und 200°C, vorzugsweise zwischen 20 und 180°C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 mol der Verbindung (II) 1 bis 15 mol, vorzugsweise 1 bis 6 mol der Verbindung (III) ein.

Freie Aminogruppen können während der Umsetzung durch eine geeignete Aminoschutzgruppe, z. B. durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester können auch durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten werden.

Die Herstellung der Säureadditionssalze der erfindungsgemäßen Verbindungen erfolgt in üblicher Weise z.B. durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden.

Säureadditionssalze der erfindungsgemäßen Verbindungen können auch dadurch erhalten werden, daß man einen Carbonsäureester der Formel (I) mit R¹ z.B. gleich Methyl oder Ethyl mit ausreichender Menge der entsprechenden Säure zu einer Carbonsäure der Formel (I) mit R¹ gleich Wasserstoff verseift und das Säureadditionssalz isoliert

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Alkali- oder Erdalkalisalze der erfindungsgemäßen Carbonsäuren können auch dadurch erhalten werden, daß man einen Carbonsäureester der Formel (I) mit R¹ z.B. gleich Methyl oder Ethyl mit einer ausreichenden Menge Alkali- oder Erdalkalilauge verseift und das entsprechende Alkali- oder Erdalkalisalz isoliert.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung vor allem auf resistente Keime und Mycoplasmen aus.

Gegenüber Bakterien, die gegenüber vergleichbare Substanzen als weniger empfindlich eingestuft werden, insbesondere resistenten Staphylococcus aureus und E. coli, zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektionsund oral verabreichbare Lösungen, Suspensionen und Emulsionen, ferner Pasten, Salben, Gele, Cremes, Lotions, Puder und Sprays genannt.

Die Wirkstoffe eignen sich bei günstiger Warmblütertoxizität bevorzugt zur Bekämpfung von bakteriellen Erkrankungen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säuretiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch fiir prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die minimalen Hemmkonzentrationen (MHK) der erfindungsgemäßen Verbindungen wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war. Die Erfassung der MHK-Werte für Mycoplasmen erfolgte mikroskopisch nach einer Inkubationszeit von 5 bis 7 Tagen.

In der nachstehenden Tabelle sind die MHK-Werte einer der erfindungsgemäßen Verbindungen im Vergleich zu Enrofloxacin als Referenzverbindung aufgeführt.

**Tabelle 1:**

| MHK-Werte (µg/ml) | | | |
|---|---|---|---|
| Spezies | Stamm | Bsp.1 | Enrofloxacin |
| | | | |
| E. coli | Ec 9658 | 0,004 | 0,03 |
| Salmonella spp. | S 9659 | 0,06 | 0,5 |
| Pseudomonas aeruginosa | P 9503 | 0,25 | 1,0 |
| Bordetella bronchiseptica | B 9610 | 0,125 | 0,5 |
| Actinobacillus pleuropneumoniae | App 06/94 | 0,008 | 0,06 |
| Staphylococcus aureus | St 2941 | 0,015 | 0,125 |
| Streptococcus agalactiae | Scc 9549 | 0,03 | 0,5 |
| Streptococcus suis | Scc 9593 | 0,06 | 0,5 |
| Actinomyces pyogenes | Am 9602 | 0,25 | 1,0 |
| Mycoplasma bovis | M 9548 | 0,015 | 0,25 |
| Mycoplasma iowae | M 95144 | 0,06 | 0,5 |
| Mycoplasma hyorhinis | M 9557 | 0,004 | 0,06 |
| Fusobacterium necrophorum | Ao 9620 | 0,03 | 4,0 |
| Clostridium perfringens | Cl 9606 | 0,25 | 4,0 |

Die verbesserten pharmakokinetischen Eigenschaften der erfindungsgemäßen Substanz aus Beispiel 1 konnte gegenüber der Referenzverbindung Enrofloxacin in einer Serumkinetik-Studie an Hunden gezeigt werden. Die Substanzen wurden in einer Dosierung von 5 mg/kg Körpergewicht an jeweils 6 Hunden der Rasse Beagle geprüft. Dabei kamen die Applikationsarten intravenös (i. v.), intramuskulär (i. m.) und oral in einer einfachen Cross-Over-Anordnung zur Anwendung. Bei allen Applikationsarten konnte gezeigt werden, daß mit der erfindungsgemäßen Substanz aus Beispiel 1 ein höherer Spitzenwert der Serumkonzentration (Cₘₐₓ), eine größere Halbwertszeit (t_{1/2}) und eine längere Verweildauer (MRT) erreicht wird und somit eine größere Substanzmenge im Organismus zur Verfügung steht (Fläche unter der Serumspiegelkurve, AUC₀₋₂₄).

**Tabelle 2:**

| Pharmakokinetische Daten | | | | | | |
|---|---|---|---|---|---|---|
| | Beispiel 1 | | | Enrofloxacin | | |
| | i.v. | i.m. | oral | i.v. | i.m. | oral |
| Cₘₐₓ [µg/ml] | / | 2,4 | 1,3 | / | 2,1 | 1,0 |
| t_{1/2} [h] | 10,5 | 6,9 | 9,5 | 3,7 | 2,9 | 4,3 |
| MRT [h] | 11,5 | 8,5 | 14,7 | 4,2 | 3,9 | 7,3 |
| AUC₀₋₂₄ [µg*h/ml) | 20,0 | 19,8 | 17,4 | 10,4 | 8,4 | 7,0 |

Die gegenüber Enrofloxacin verbesserte Aufnahmeakzeptanz der erfindungsgemäßen Verbindung aus Beispiel 1 konnte durch Verfütterung an 20 kg schwere Läuferschweine in einer Dosierung von 150 ppm nachgewiesen werden. Während die Tiere die Aufnahme Enrofloxacin-haltigen Futters in Dosierungen von bereits 50 ppm verweigern, wurde die erfindungsgemäße Substanz problemlos innerhalb von 15 Minuten restlos aufgenommen.

### Herstellung der Wirkstoffe

### Beispiel 1

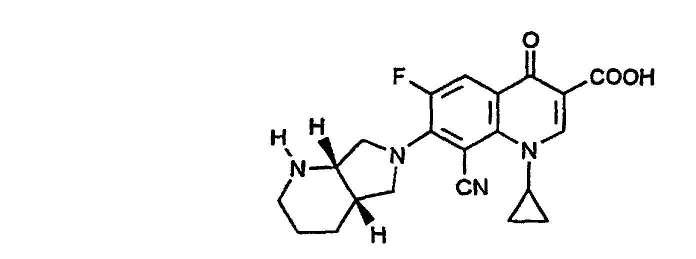

### 8-Cyan-1-cyclopropyl-7-((1S,6S)-2,8-diazabicyclo[4.3.0]nonan-8-yl)-6-fluor-1,4-dihydro-4-oxo-3-chinolincarbonsäure

690 mg (2.25 mmol) 7-Chlor-8-cyan-1-cyclopropyl-6-fluor-1,4-dihydro-4-oxo-3-chinolincarbonsäure werden mit 312 mg (2.47 mmol) (1S,6S)-2,8-Diazabicyclo-[4.3.0]nonan und 504 mg (4.50 mmol) 1,4-Diazabicyclo[2.2.2]octan (DABCO) in einem Gemisch aus 6.6 ml Dimethylformamid und 6.6 ml Acetonitril über Nacht bei Raumtemp. gerührt. Alle flüchtigen Komponenten werden i. Vak. entfernt, der Rückstand in Wasser aufgenommen und die resultierende Lösung mit verd. Salzsäure auf pH 7 eingestellt. Der entstehende Niederschlag wird abgesaugt, das Filtrat mit Dichlormethan extrahiert, die vereinigten organischen Phasen mit Natriumsulfat getrocknet und i. Vak. eingeengt.
Ausbeute: 650 mg (73 %)
Schmelzpunkt: 246-248°C (Zers.)

### Beispiel 2

### 8-Cyan-1-cyclopropyl-7-((1S,6S)-2,8-diazabicyclo[4.3.0]nonan-8-yl)-6-fluor-1,4-dihydro-4-oxo-3-chinolincarbonsäure-Hydrochlorid

5.00 g (12.6 mmol) 8-Cyan-1-cyclopropyl-7-((1S,6S)-2,8-diazabicyclo[4.3.0]nonan-8-yl)-6-fluor-1,4-dihydro-4-oxo-3-chinolincarbonsäure werden zwei Stunden in 95 ml 4 N Salzsäure/ Dioxan (1:1) bei 60°C gerührt. Das Reaktionsgemisch wird im Vakuum eingeengt und der Rückstand aus Ethanol umkristallisiert.
Ausbeute: 4.45 g (82 % der Theorie)
Schmelzpunkt: 280°C (unter Zersetzung)

### Beispiel 3

### 8-Cyan-1-cyclopropyl-7-((1S,6S)-2,8-diazabicyclo[4.3.0]nonan-8-yl)-6-fluor-1,4-dihydro-4-oxo-3-chinolincarbonsäure-Mesylat

250 mg (0.63 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure werden in 2 ml Wasser gelöst und mit einem Äquivalent Methansulfonsäure versetzt. Die Lösung wird 30 min bei Raumtemp. gerührt und dann auf 20 ml Ethanol gegeben. Der resultierende Niederschlag wird abgesaugt und anschließend getrocknet.
Ausbeute: 201 mg (65 % der Theorie)
Schmelzpunkt: 118-124°C

### Beispiel 4

### 8-Cyan-1-cyclopropyl-7-((1S,6S)-2,8-diazabicyclo[4.3.0]nonan-8-yl)-6-fluor-1,4-dihydro-4-oxo-3-chinolincarbonsäure-Tosylat

250 mg (0.63 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure werden in 2 ml Wasser gelöst und mit einem Äquivalent Toluolsulfonsäure versetzt. Die Lösung wird 30 min bei Raumtemp. gerührt und dann auf 20 ml Ethanol gegeben. Der resultierende Niederschlag wird abgesaugt und anschließend getrocknet.
Ausbeute: 309 mg (86 % der Theorie)
Schmelzpunkt: 222-230°C

### Beispiel 5

### 8-Cyan-1-cyclopropyl-7-((1S,6S)-2,8-diazabicyclo[4.3.0]nonan-8-yl)-6-fluor-1,4-dihydro-4-oxo-3-chinolincarbonsäure-Trifluoracetat

200 mg (0.50 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure werden in 3 ml Ethanol suspendiert und mit einem Äquivalent Trifluoressigsäure versetzt. Die entstandene Lösung wird 30 min unter Rückfluß erhitzt und anschließend abgekühlt. Der resultierende Niederschlag wird abgesaugt und mit Ether gewaschen.
Ausbeute: 208 mg (81 % der Theorie)
Schmelzpunkt: 170-178°C

### Beispiel 6

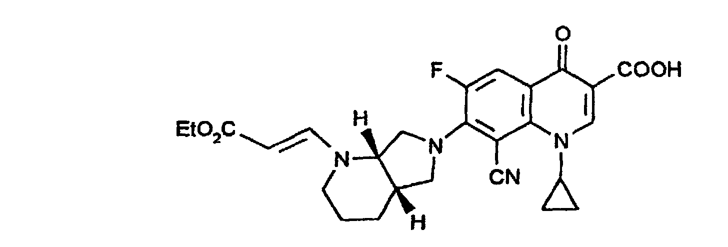

### 8-Cyan-1-cyclopropyl-7-[(1S,6S)-2-(2-ethoxycarbonyl-vinyl)-2,8-diazabicyclo[4.3.0]nonan-8-yl]-6-fluor-1,4-dihydro-4-oxo-3-chinolincarbonsäure

400 mg (1.01 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure und 1.03 ml (10.1 mmol) Propiolsäureethylester werden in 7.5 ml Methylglykol 1 h auf 120°C erhitzt. Die Reaktionslösung wird i. Vak. eingeengt, der Rückstand mit Wasser verrührt und abgesaugt. Das resultierende Rohprodukt wird aus Ethanol umkristallisiert.
Ausbeute: 302 mg (61 % der Theorie)
Schmelzpunkt: 180-182°C

### Beispiel 7

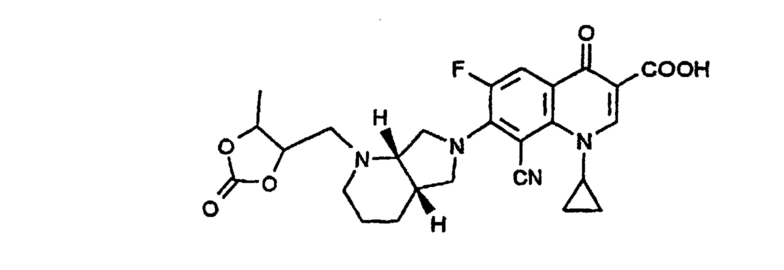

### 8-Cyan-1-cyclopropyl-7-[(1S,6S)-2-(5-methyl-2-oxo-1,3-dioxol-4-yl)-methyl-2,8-diazabicyclo[4.3.0]nonan-8-yl]-6-fluor-1,4-dihydro-4-oxo-3-chinolincarbonsäure

100 mg (0.25 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure, 59 mg (0.30 mmol) 4-Bromomethyl-5-methyl-1,3-dioxol-2-on und 30 mg Kaliumhydrogencarbonat werden in 2 ml Dimethylformamid 30 min auf 140°C erhitzt. Die Reaktionslösung wird i. Vak. eingeengt, der Rückstand in Dichlormethan aufgenommen und mit Wasser gewaschen. Die organische Phase wird über Magnesiumsulfat getrocknet und i. Vak. eingeengt. Der resultierende Rückstand wird mit Wasser verrührt, abgesaugt und getrocknet.
Ausbeute: 99 mg ( 77 % der Theorie)
Schmelzpunkt: 175°C (unter Zersetzung)

### Beispiel 8

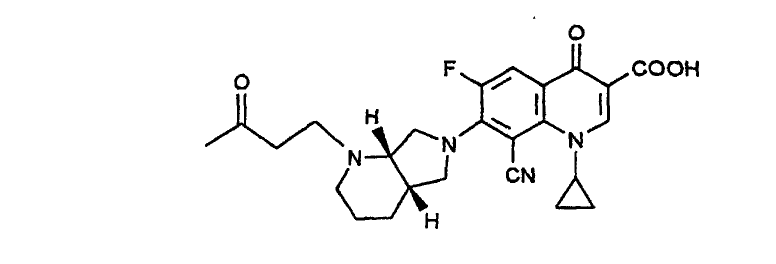

### 8-Cyan-1-cyclopropyl-7-[(1S,6S)-2-(3-oxo-butyl)-2,8-diazabicyclo[4.3.0]nonan-8-yl]-6-fluor-1,4-dihydro-4-oxo-3-chinolincarbonsäure

300 mg (0.76 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure und 0.63 ml (7.6 mmol) Methylvinylketon werden in 5 ml Methylglykol 2 h unter Rückfluß erhitzt. Die Reaktionslösung wird i. Vak. eingeengt, der Rückstand mit Wasser verrührt und abgesaugt.
Ausbeute: 245 mg (69 % der Theorie)
Schmelzpunkt: 158-160°C

### Beispiel 9

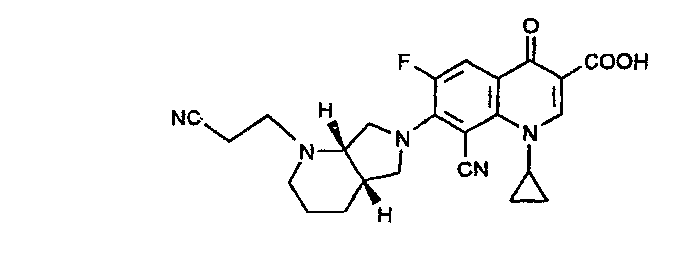

### 8-Cyan-1-cyclopropyl-7-[(1S,6S)-2-(2-cyanethyl)-2,8-diazabicyclo[4.3.0]nonan-8-yl]-6-fluor-1,4-dihydro-4-oxo-3-chinolincarbonsäure

400 mg (1.01 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure und 1.03 ml (10.1 mmol) Acrylsäurenitril werden in 7.5 ml Methylglykol 1 h auf 120°C erhitzt. Die Reaktionslösung wird i. Vak. eingeengt, der Rückstand mit Wasser verrührt und abgesaugt Das resultierende Rohprodukt wird aus Ethanol umkristallisiert.
Ausbeute: 136 mg (91 % der Theorie)
Schmelzpunkt: 250°C

### Beispiel 10

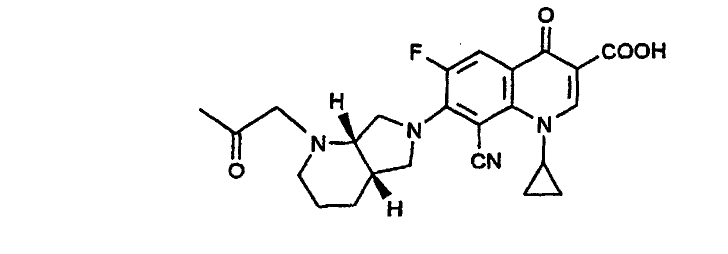

### 8-Cyan-1-cyclopropyl-7-[(1S,6S)-2-(2-oxopropyl)-2,8-diazabicyclo[43.0]nonan-8-yl]-6-fluor-1,4-dihydro-4-oxo-3-chinolincarbonsäure

Analog zum Beispiel 7 wird bei der Umsetzung mit Chloraceton die Titelverbindung erhalten.
Schmelzpunkt: 74-75°C

### Beispiel 11

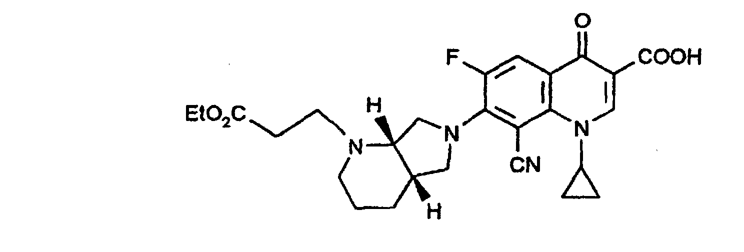

### 8-Cyan-1-cyclopropyl-7-[(1S,6S)-2-(2-ethoxycarbonyl-ethyl)-2,8-diazabicyclo[4.3.0]nonan-8-yl]-6-fluor-1,4-dihydro-4-oxo-3-chinolincarbonsäure

Analog zum Beispiel 8 wird bei der Umsetzung mit Acrylsäureethylester die Titelverbindung erhalten.
Schmelzpunkt: 148-150°C

### Beispiel 12

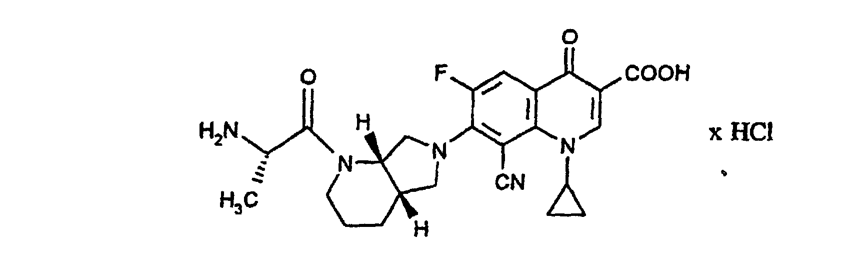

### 8-Cyan-1-cyclopropyl-7-[(1S,6S)-2-((S)-alanyl)-2,8-diazabicyclo[4.3.0]nonan-8-yl]-6-fluor-1,4-dihydro-4-oxo-3-chinolincarbonsäure-Hydrochlorid

250 mg (0.63 mmol) 8-Cyan-1-cyclopropyl-7-((1S,6S)-2,8-diazabicyclo[4.3.0]-nonan-8-yl)-6-fluor-1,4-dihydro-4-oxo-3-chinolincarbonsäure, 150 mg (0.69 mmol) N-tert.-Butyloxycarbonyl-L-alanin-N-carboxyanhydrid und 12.5 mg N,N-Dimethylaminopyridin werden in 7.5 ml Dimethylformamid gelöst. Die Lösung wird 3 h bei Raumtemp. gerührt und dann i. Vak. eingeengt. Der Rückstand wird mit 20 ml 4 N Salzsäure/Dioxan (1:1) versetzt und 3 h auf 60°C erhitzt. Die Reaktionsmischung wird i. Vak. eingeengt und anschließend der resultierende Rückstand aus Acetonitril umkristallisiert.
Ausbeute: 164 mg (52 % der Theorie)
Schmelzpunkt: 245°C (unter Zersetzung)

### Beispiel 13

### 8-Cyan-1-cyclopropyl-7-[(1S,6S)-2-((R)-alanyl)-2,8-diazabicyclo[4.3.0]nonan-8-yl]-6-fluor-1,4-dihydro-4-oxo-3-chinolincarbonsäure-Hydrochlorid

Analog zum Beispiel 12 wird bei der Umsetzung mit N-tert.-Butyloxycarbonyl-Dalanin-N-carboxyanhydrid die Titelverbindung erhalten.
Schmelzpunkt: 213°C (unter Zersetzung)

### Beispiel 14

### 8-Cyan-1-cyclopropyl-7-[(1S,6S)-2-((S)-valinyl)-2,8-diazabicyclo[4.3.0]nonan-8-yl]-6-fluor-1,4-dihydro-4-oxo-3-chinolincarbonsäure-Hydrochlorid

Analog zum Beispiel 12 wird bei der Umsetzung mit N-tert.-Butyloxycarbonyl-Lvalin-N-carboxyanhydrid die Titelverbindung erhalten.
Schmelzpunkt: 255°C (unter Zersetzung)

### Beispiel 15

### 8-Cyan-1-cyclopropyl-7-[(1S,6S)-2-((S)-phenylalanyl)-2,8-diazabicyclo[4.3.0]nonan-8-yl]-6-fluor-1,4-dihydro-4-oxo-3-chinolincarbonsäure-Hydrochlorid

Analog zum Beispiel 12 wird bei der Umsetzung mit N-tert.-Butyloxycarbonyl-Lphenylalanin-N-carboxyanhydrid die Titelverbindung erhalten.
Schmelzpunkt: 230°C (unter Zersetzung)

### Beispiel 16

### 8-Cyan-1-cyclopropyl-7-[(1S,6S)-2-((S)-leucinyl)-2,8-diazabicyclo[4.3.0]nonan-8-yl]-6-fluor-1,4-dihydro-4-oxo-3-chinolincarbonsäure-Hydrochlorid

Analog zum Beispiel 12 wird bei der Umsetzung mit N-tert.-Butyloxycarbonyl-Lleucin-N-carboxyanhydrid die Titelverbindung erhalten.
Schmelzpunkt: 270-274°C (unter Zersetzung)

### Beispiel 17

### 8-Cyano-1-cyclopropyl-7-[(1S,6S)-2,8-diazabicyclo[4.3.0]nonan-8-yl]-6-fluor-1,4-dihydro-4-oxo-3-chinolincarbonsäuremethylester

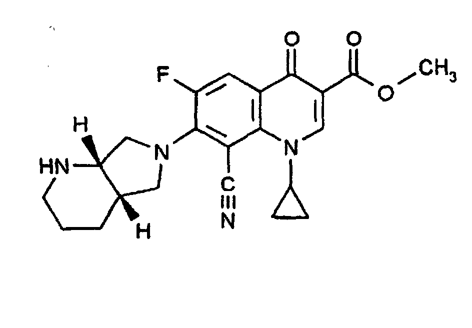

200 mg (0,625 mmol) 8-Cyano-1-cyclopropyl-7-chlor-6-fluor-1,4-dihydro-4-oxo-3-chinolincarbonsäuremethylester, 86 mg (0,683 mmol) (1S,6S)-2,8-Diazabicyclo-[4.3.0]nonan und 150 mg (1,34 mmol) 1,4-Diazabicyclo[2.2.2]octan werden in 6 ml Acetonitril 48 Stunden bei Raumtemperatur gerührt. Danach wird eingedampft und zwischen 15 ml Chloroform und 20 ml gesättigter Natriumcarbonat-Lösung verteilt. Die organische Phase wird abgetrennt, die wäßrige mit Chloroform nachextrahiert und die vereinigten Extrakte nach Trocknung mit Natriumsulfat eingedampft. Zur Reinigung wird an Kieselgel mit Essigester/Ethanol/25%iger wäßriger Ammoniak-Lösung chromatographiert.
Ausbeute: 140 mg
Schmelzpunkt: 231°C (unter Zersetzung)

### Beispiel 18

### 8-Cyano-1-cyclopropyl-7-[(1S,6S)-2,8-diazabicyclo(4.3.0)nonan-8-yl]-6-fluor-1,4-dihydro-4-oxo-3-chinolincarbonsäureethylester

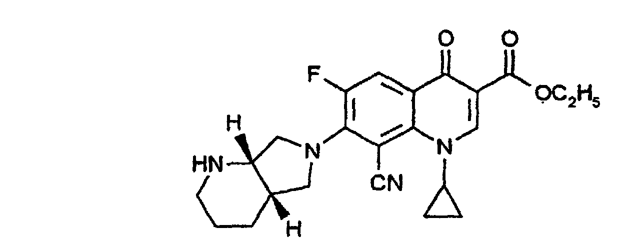

14,32 g (45 mmol) 8-Cyan-1-cyclopropyl-6,7-difluor-1,4-dihydro-4-oxo-3-chinolincarbonsäureethylester, 6,31 g (50 mmol) (1S,6S)-2,8-Diazabicyclo[4.3.0]nonan und 10,22 g (101 mmol) Triethylamin werden in 270 ml Acetonitril 4 Stunden unter Rückfluß gekocht. Man läßt das Reaktionsgemisch einige Stunden bei Raumtemperatur stehen, saugt den auskristallisierten Feststoff ab, wäscht mit Acetonitril nach und trocknet. Man erhält 15,6 g beigen Feststoff (82 % der Theorie).
Schmelzpunkt: 209 bis 210°C

### Beispiel 19

### 8-Cyan-1-cyclopropyl-7-[(1S,6S)-2,8-diazabicyclo[4.3.0]nonan-8-yl]-6-fluor-1,4-dihydro-4-oxo-3-chinolincarbonsäure-Natriumsalz

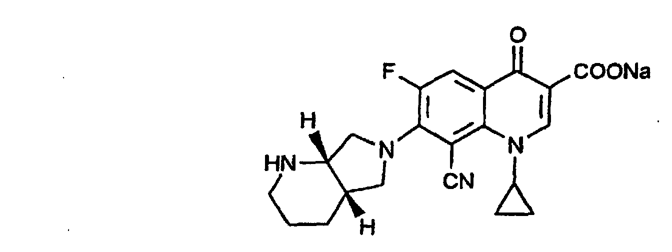

2,12 g (5 mmol) 8-Cyan-1-cyclopropyl-7-[(1S,6S)-2,8-diazabicyclo[4.3.0]nonan-8-yl]-6-fluor-1,4-dihydro-4-oxo-3-chinolincarbonsäure-ethylester und 0,21 g (5,2 mmol) Natriumhydroxid werden in 10 ml Ethanol 2 Stunden zum Rückfluß erhitzt. Der überwiegende Teil der Ethanol wird dann im Vakuum abgezogen. Der Rückstand wird mit Hexan vermehrt und der resultierende Feststoff abgesaugt und getrocknet. Man erhält 2,07 g beigen Feststoff (98,9 % der Theorie).
Schmelzpunkt: 235°C (unter Zersetzung)

### Beispiel 20

### Ethyl-8-cyano-1-cyclopropyl-6,7-difluoro-1,4-dihydro-4-oxo-3-quinolinecarboxylat

### Stufe a: Methyl-3-bromo-2,4,5-trifluorbenzoat

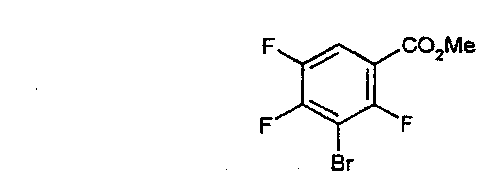

772 g 3-Bromo-2,3,5-trifluoro-benzoyl-fluorid werden unter Eiskühlung in 1460 ml Methanol und 340 g Triethylamin zugetropft. Die Mischung wird 1 Stunde bei Raumtemperatur gerührt Das Reaktionsgemisch wird im Vakuum eingeengt und der Rückstand in Wasser und Methylenchlorid aufgenommen; die beiden Phasen werden getrennt und die wässrige Phase erneut mit Methylenchlorid behandelt. Die Methylenchloridphasen werden über Na₂CO₃ getrocknet und im Vakuum eingeengt Der Rückstand wird im Vakuum destilliert. Ausbeute: 752,4 g; Kochpunkt 122°C/20 mbar.

### Stufe b: Methyl-3-cyano-2,4,5-trifluorobenzoat

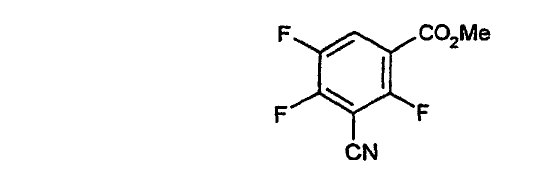

Eine Mischung aus 269 g Methyl-2-bromo-2,4,5-trifluorobenzoat, 108 g auf Kupfer(I)cyanid und 400 ml Dimethylformamid wird 5 Stunden am Rückfluß gekocht. Alle flüchtigen Komponenten werden im Vakuum abdestilliert. Fraktionierte Destillation ergibt 133 g der gewünschten Verbindung vom Kochpunkt 88-89°C/0,01 mbar.

### Stufe c: 3-Cyano-2,4,5-trifluoro-benzoesäure

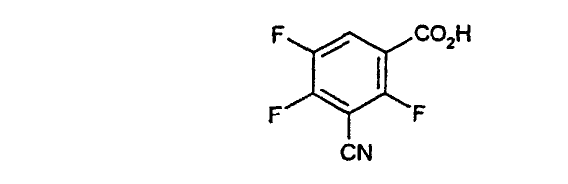

Eine Lösung von 156 g Methyl-3-cyano-2,4,5-trifluoro-benzoat in einer Mischung von 960 ml Eisessig, 140 ml Wasser und 69 ml aus H₂SO₄ conc. wird 8 Std. am Rückfluß gekocht. Die Essigsäure wird abdestilliert und der Rückstand mit Wasser aufgenommen. Der Niederschlag wird abfiltriert, gewaschen und getrocknet.
Ausbeute: 118,6 g weißes Pulver
Schmelzpunkt: 187-190

### Stufe d: 3-Cyano-2,4,5-trifluoro-benzoyl-chlorid

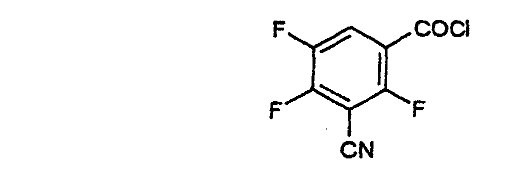

111 g 3-Cyano-2,4,5-trifluor-benzoesäure, 84 g aus Oxalylchlorid und einige Tropfen Dimethylformamid in 930 ml trockenes Methylenchlorid werden bei Zimmertemperatur 5 Std. gerührt. Die Mischung wird eingeengt und der Rückstand am Vakuum destilliert.
Ausbeute: 117,6 g eines gelben Öls

### Stufe e: Ethyl-2-(3-Cyano-2,4,5-trifluoro-benzoyl)-3-dimethylaminoacrylat

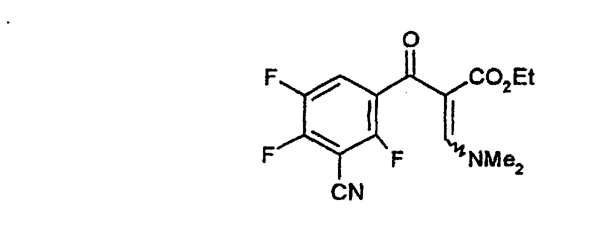

Eine Lösung von 55 g 3-Cyano-2,4,5-trifluoro-benzoylchlorid in 50 ml Toluol wird in einer Lösung von 36,5 g Ethyl-3-dimethylamino-acrylat und 26,5 g Triethylamine in 140 ml Toluol bei Temperatur zwischen 50 und 55°C zugetropft. Die Reaktionsmischung wird 2 Stunden bei 50°C gerührt und dann im Vakuum eingeengt. Das rohe Produkt wird ohne weitere Reinigung in der nächsten Stufe eingesetzt.

### Stufe f: Ethyl-2-(3-Cyano-2,4,5-trifluoro-benzoyl)-3-cycloppropylaminoacrylat

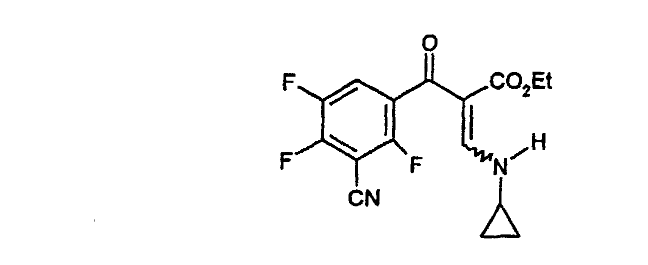

30 g Eisessig werden bei 20°C zum Rohprodukt der Stufe zugetropft. Dann werden 15,75 g Cyclopropylamin in 30 ml Toluol zugetropft. Die Mischung wird 1 Stunde bei 30°C gerührt. Dann werden 200 ml Wasser zugegeben und weitere 15 Minuten gerührt. Die organische Phase wird abgetrennt, mit 100 ml Wasser extrahiert, über Na₂CO₃ getrocknet und im Vakuum eingeengt. Das rohe Produkt wird ohne weitere Reinigung in der nächsten Stufe eingesetzt.

### Stufe g: Ethyl-8-cyano-1-cyclopropyl-6,7-difluoro-1,4-dihydro-4-oxoquinoline-3-carboxylat

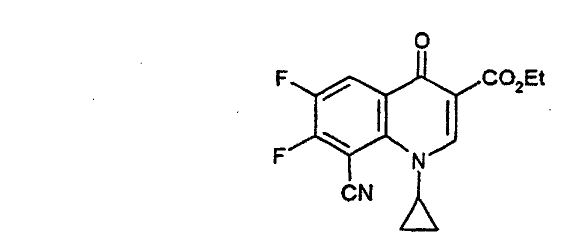

Eine Mischung des rohen Produkts aus Stufe f, 27,6 g K₂CO₃ und 80 ml DMF werden bei Raumtemperatur 16 Stunden gerührt. Dann wird das Reaktionsgemisch in 750 ml/Eiswasser gegeben, der Niederschlag abfiltriert, mit 80 ml kaltem Methanol gewaschen und getrocknet.
Ausbeute: 47 g
Schmelzpunkt 209-211°C.

## Patentansprüche

1. Gegebenenfalls substituierte 8-Cyan-1-cyclopropyl-7-(2,8-diazabicyclo[4.3.0]-nonan-8-yl)-6-fluor-1,4-dihydro-4-oxo-3-chinolincarbonsäuren und ihre Derivate der allgemeinen Formel (I) 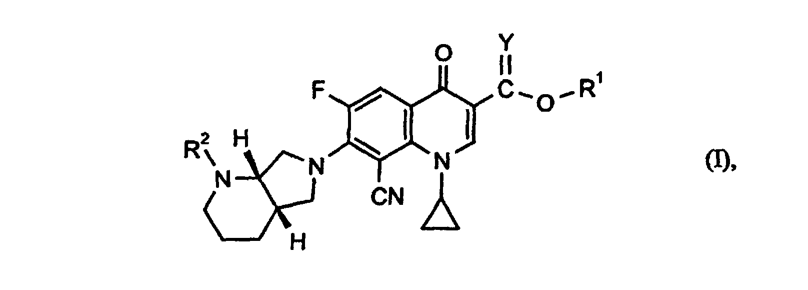 in welcher
R¹ für Wasserstoff, gegebenenfalls durch Hydroxy, Methoxy, Amino, Methylamino oder Dimethylamino substituiertes C₁-C₄-Alkyl oder (5-Methyl-2-oxo-1,3-dioxol-4-yl)-methyl steht,
R² für Wasserstoff, Benzyl, C₁-C₃-Alkyl, (5-Methyl-2-oxo-1,3-dioxol-4-yl)-methyl, Reste der Strukturen -CH=CH-COOR³, -CH₂CH₂COOR³, -CH₂CH₂CN, -CH₂CH₂COCH₃, -CH₂COCH₃, in denen R³ für Methyl oder Ethyl steht, oder einen Rest der allgemeinen Struktur R⁴-(NH-CHR⁵-CO)ₙ- steht, in der R⁴ für Wasserstoff, C₁-C₃-Alkyl oder den Rest -COO-tert.-Butyl steht, R⁵ für Wasserstoff, C₁-C₄-Alkyl, Hydroxyalkyl, Aminoalkyl, Thioalkyl, Carboxyalkyl oder Benzyl steht und n = 1 oder 2 ist,
und
Y für Sauerstoff steht,
und deren pharmazeutisch verwendbaren Hydrate und Säureadditionssalze sowie die Alkali, Erdalkali-, Silber- und Guanidiniumsalze der zugrundeliegenden Carbonsäure.

2. Verfahren zur Herstellung von gegebenenfalls substituierten 8-Cyan-1-cyclopropyl-7-(2,8-diazabicyclo[4.3.0]nonan-8-yl)-6-fluor-1,4-dihydro-4-oxo-3-chinolincarbonsäuren, **dadurch gekennzeichnet daß** man Verbindungen der Formel (II) 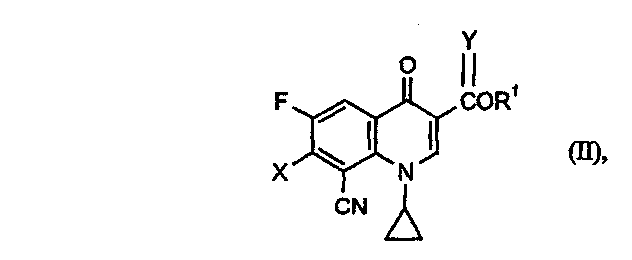 in welcher
R¹ und Y die in Anspruch 1 angegebene Bedeutung hat und
X für Halogen, insbesondere für Fluor oder Chlor steht,
mit 2,8-Diazabicyclo[4.3.0]nonanen der Formel (III) 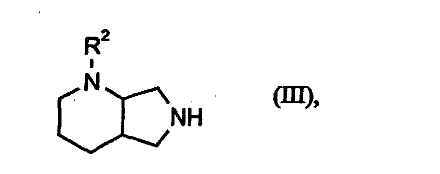 in welcher
R² die oben angegebene Bedeutung hat
gegebenenfalls in Gegenwart von Säurebindemitteln umsetzt; gegebenenfalls wird anschließend der Carbonsäureester gespalten; gegebenenfalls werden anschließend Verbindungen der Formel (I), in welcher R² flir Wasserstoff steht, N-alkyliert, N-alkenyliert oder N-acyliert.

3. Verbindungen der Formel (I) gemäß Anspruch 1, in welcher
R¹ für Wasserstoff, gegebenenfalls durch Hydroxy, Methoxy, Amino, Methylamino oder Dimethylamino substituiertes C₁-C₄-Alkyl oder (5-Methyl-2-oxo-1,3-dioxol-4-yl)-methyl steht,
R² für Wasserstoff, Benzyl, C₁-C₃-Alkyl, (5-Methyl-2-oxo-1,3-dioxol-4-yl)-methyl, Reste der Strukturen -CH=CH-COOR³, -CH₂CH₂COOR³, -CH₂CH₂CN, -CH₂COCH₃, in denen R³ für Methyl oder Ethyl steht, oder einen Rest der allgemeinen Struktur R⁴-(NH-CHR⁵-CO)ₙ- steht, in der R⁴ für Wasserstoff, C₁-C₃-Alkyl oder den Rest -COO-tert-Butyl steht, R⁵ für Wasserstoff, C₁-C₄-Alkyl, Hydroxyalkyl, Aminoalkyl, Thioalkyl oder Benzyl steht und n = 1 oder 2 ist,
und
Y für Sauerstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

4. Verbindungen der Formel (I) gemäß Anspruch 1, in welcher
R¹ für Wasserstoff, C₁-C₄-Alkyl oder (5-Methyl-2-oxo-1,3-dioxol-4-yl)methyl steht,
R² für Wasserstoff, Methyl, (5-Methyl-2-oxo-1,3-dioxol-4-yl)-methyl, Reste der Strukturen -CH=CH-COOR³, -CH₂CH₂COOR³, -CH₂CH₂CN, -CH₂COCH₃, in denen R³ für Methyl oder Ethyl steht, oder einen Rest der allgemeinen Struktur R⁴-(NH-CHR⁵-CO)ₙ- steht, in der R⁴ für Wasserstoff, C₁-C₃-Alkyl oder den Rest -COO-tert.-Butyl steht, R⁵ für Wasserstoff, C₁-C₄-Alkyl, Hydroxyalkyl, Aminoalkyl, Thioalkyl oder Benzyl steht und n = 1 oder 2 ist,
und
Y für Sauerstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

5. 8-Cyan-1-cyclopropyl-7-(2,8-diazabicyclo-[4.3.0]nonan-8-yl)-6-fluor-1,4-dihydro-4-oxo-3-chinolincarbonsäure und deren Ester, wie sie in den Ansprüchen 1, 3 und 4 für Verbindungen der Formel (I) aufgeführt sind.

6. Arzneimittel enthaltend Verbindungen der Formel (I) gemäß Anspruch 1.

7. Verwendung von Verbindungen der Formel (I) gemäß Anspruch 1 zur Herstellung von Arzneimitteln.

8. Verwendung gemäß Anspruch 7 zur Herstellung von Arzneimitteln zur Bekämpfung von gramnegativen und grampositiven Bakterien sowie von bakterienähnlichen Mikroorganismen.

## Claims

1. Optionally substituted 8-cyano-1-cyclopropyl-7-(2,8-diazabicyclo[4.3.0]-nonan-8-yl)-6-fluoro-1,4-dihydro-4-oxo-3-quinolinecarboxylic acids and their derivatives of the general formula (I) 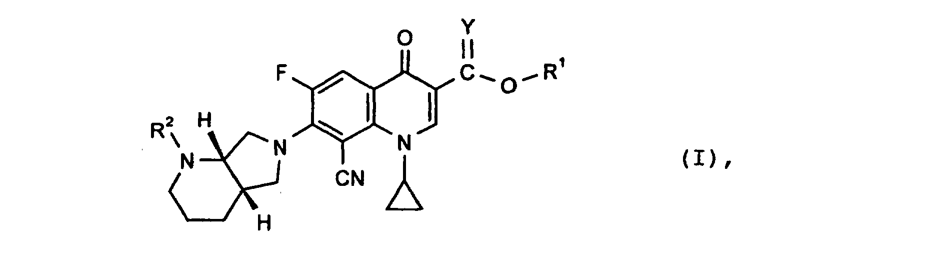 in which
R¹ represents hydrogen, C₁-C₄-alkyl which is optionally substituted by hydroxyl, methoxy, amino, methylamino or dimethylamino, or (5-methyl-2-oxo-1,3-dioxol-4-yl)methyl,
R² represents hydrogen, benzyl, C₁-C₃-alkyl, (5-methyl-2-oxo-1,3-dioxol-4-yl)methyl, radicals having the structures -CH=CH-COOR³, -CH₂CH₂COOR³, -CH₂CH₂CN, -CH₂CH₂COCH₃ or -CH₂COCH₃, in which R³ represents methyl or ethyl, or a radical of the general structure R⁴-(NH-CHR⁵-CO)ₙ-, in which R⁴ represents hydrogen, C₁-C₃-alkyl or the radical -COO-tert-butyl, R⁵ represents hydrogen, C₁-C₄-alkyl, hydroxyalkyl, aminoalkyl, thioalkyl, carboxyalkyl or benzyl and n is 1 or 2,
and
Y is oxygen,
and pharmaceutically usable hydrates and acid addition salts thereof, as well as the alkali metal, alkaline earth metal, silver and guanidinium salts of the carboxylic acids on which the compounds are based.

2. Process for the preparation of optionally substituted 8-cyano-1-cyclopropyl-7-(2,8-diazabicyclo[4.3.0]nonan-8-yl)-6-fluoro-1,4-dihydro-4-oxo-3-quinolinecarboxylic acids, **characterized in that** compounds of the formula (II) 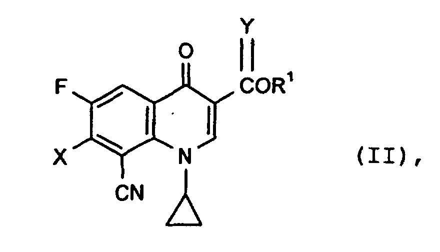 in which
R¹ and Y have the meaning given in Claim 1 and
X represents halogen, in particular fluorine or chlorine,
are reacted with 2,8-diazabicyclo[4.3.0]nonanes of the formula (III) 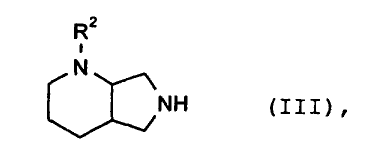 in which
R² has the abovementioned meaning,
if appropriate in the presence of acid-binding agents; if appropriate, the carboxylic acid ester is then cleaved; if appropriate, compounds of the formula (I) in which R² represents hydrogen are then N-alkylated, N-alkenylated or N-acylated.

3. Compounds of the formula (I) according to Claim 1, in which
R¹ represents hydrogen, C₁-C₄-alkyl which is optionally substituted by hydroxyl, methoxy, amino, methylamino or dimethylamino, or (5-methyl-2-oxo-1,3-dioxol-4-yl)methyl,
R² represents hydrogen, benzyl, C₁-C₃-alkyl, (5-methyl-2-oxo-1,3-dioxol-4-yl)methyl, radicals of the structures -CH=CH-COOR³, -CH₂CH₂COOR³, -CH₂CH₂CN or -CH₂COCH₃, in which R³ represents methyl or ethyl, or a radical of the general structure R⁴-(NH-CHR⁵-CO)ₙ-, in which R⁴ represents hydrogen, C₁-C₃-alkyl or the radical -COO-tert-butyl, R⁵ represents hydrogen, C₁-C₄-alkyl, hydroxyalkyl, aminoalkyl, thioalkyl or benzyl and n is 1 or 2,
and
Y represents oxygen,
and pharmaceutically usable hydrates and acid addition salts thereof, as well as the alkali metal, alkaline earth metal, silver and guanidinium salts of the carboxylic acids on which the compounds are based.

4. Compounds of the formula (I) according to Claim 1, in which
R¹ represents hydrogen, C₁-C₄-alkyl or (5-methyl-2-oxo-1,3-dioxol-4-yl)methyl,
R² represents hydrogen, methyl, (5-methyl-2-oxo-1,3-dioxol-4-yl)methyl, radicals of the structures -CH=CH-COOR³, -CH₂CH₂COOR³, -CH₂CH₂CN or -CH₂COCH₃, in which R³ represents methyl or ethyl, or a radical of the general structure R⁴-(NH-CHR⁵-CO)ₙ-, in which R⁴ represents hydrogen, C₁-C₃-alkyl or the radical -COO-tert-butyl, R⁵ represents hydrogen, C₁-C₄-alkyl, hydroxyalkyl, aminoalkyl, thioalkyl or benzyl and n is 1 or 2,
and
Y represents oxygen,
and pharmaceutically usable hydrates and acid addition salts thereof, as well as the alkali metal, alkaline earth metal, silver and guanidinium salts of the carboxylic acids on which the compounds are based.

5. 8-Cyano-1-cyclopropyl-7-(2,8-diazabicyclo[4.3.0]-nonan-8-yl)-6-fluoro-1,4-dihydro-4-oxo-3-quinolinecarboxylic acid and esters thereof, as listed in Claims 1, 3 and 4 for compounds of the formula (I).

6. Compounds of the formula (I) according to Claim 1 comprising medicaments.

7. Use of compounds of the formula (I) according to Claim 1 for the preparation of medicaments.

8. Use according to Claim 7 for the preparation of medicaments for controlling Gram-negative and Gram-positive bacteria and bacteria-like microorganisms.

## Revendications

1. Acides 8-cyano-1-cyclopropyl-7-(2,8-diazabicyclo-[4.3.0]-nonan-8-yl)-6-fluoro-1,4-dihydro-4-oxo-3-quinoléinecarboxyliques le cas échéant substitués, ainsi que leurs dérivés répondant à la formule générale (I) 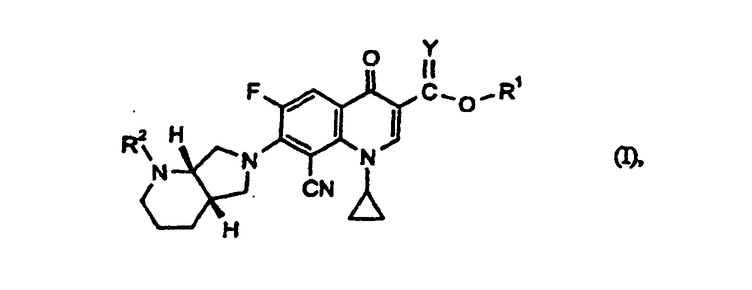 dans laquelle
R¹ représente un atome d'hydrogène, un groupe alkyle en C₁-C₄ portant, le cas échéant, un ou plusieurs substituants identiques ou différents hydroxyle, méthoxy, amino, méthylamino ou diméthylamino, ou encore un groupe (5-méthyl-2-oxo-1,3-dioxol-4-yl)méthyle,
R² représente un atome d'hydrogène, un groupe benzyle, un groupe alkyle en C₁-C₃, un groupe (5-méthyl-2-oxo-1,3-dioxol-4-yl)-méthyle, des radicaux répondant aux structures -CH=CH-COOR³, -CH₂CH₂COOR³, -CH₂CH₂CN, -CH₂CH₂COCH₃, -CH₂COCH₃, dans lesquelles R³ représente un groupe méthyle ou un groupe éthyle, ou encore un radical répondant à la structure générale R⁴-(NH-CHR⁵-CO)ₙ dans laquelle R⁴ représente un atome d'hydrogène, un groupe alkyle en C₁-C₃, ou encore le radical -COO-tert.-butyle, R⁵ représente un atome d'hydrogène, un groupe alkyle en C₁-C₄, un groupe hydroxyalkyle, un groupe aminoalkyle, un groupe thioalkyle, un groupe carboxyalkyle ou un groupe benzyle et n = 1 ou 2,
et
Y représente un atome d'oxygène,
ainsi que leurs hydrates et leurs sels d'addition d'acides pharmaceutiquement acceptables, de même que les sels de métaux alcalins, de métaux alcalino-terreux, d'argent et de guanidium de l'acide carboxylique de base.

2. Procédé pour la préparation d'acides 8-cyano-1-cyclopropyl-7-(2,8-diazabicyclo[4.3.0]-nonan-8-yl)-6-fluoro-1,4-dihydro-4-oxo-3-quinoléinecarboxyliques le cas échéant substitués, **caractérisé en ce qu'**on fait réagir des composés répondant la formule (II) 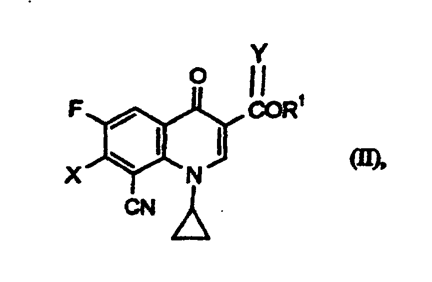 dans laquelle
R¹ et Y ont la signification indiquée à la revendication 1, et
X représente un atome d'halogène, en particulier un atome de fluor ou un atome de chlore,
avec des 2,8-diazabicyclo[4.3.0]nonanes répondant à la formule (III) 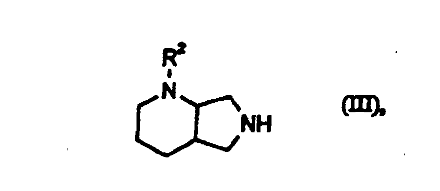 dans laquelle
R² a la signification indiquée ci-dessus
le cas échéant en présence d'agents neutralisant les acides ; le cas échéant, on soumet ensuite les esters carboxyliques à une dissociation ; le cas échéant, on soumet ensuite les composés répondant à la formule (I), dans laquelle R² représente un atome d'hydrogène, à une alkylation, à une alcénylation ou à une acylation sur l'atome d'azote.

3. Composés répondant à la formule (I), selon la revendication 1, dans lesquels
R¹ représente un atome d'hydrogène, un groupe alkyle en C₁-C₄ portant, le cas échéant, un ou plusieurs substituants identiques ou différents hydroxyle, méthoxy, amino, méthylamino ou diméthylamino, ou encore un groupe (5-méthyl-2-oxo-1,3-dioxol-4-yl)méthyle,
R² représente un atome d'hydrogène, un groupe benzyle, un groupe alkyle en C₁-C₃, un groupe (5-méthyl-2-oxo-1,3-dioxol-4-yl)-méthyle, des radicaux répondant aux structures -CH=CH-COOR³, -CH₂CH₂COOR³, -CH₂CH₂CN, -CH₂COCH₃, dans lesquelles R³ représente un groupe méthyle ou un groupe éthyle, ou encore un radical répondant à la structure générale R⁴-(NH-CHR⁵-CO)ₙ dans laquelle R⁴ représente un atome d'hydrogène, un groupe alkyle en C₁-C₃, ou encore le radical -COO-tert.-butyle, R⁵ représente un atome d'hydrogène, un groupe alkyle en C₁-C₄, un groupe hydroxyalkyle, un groupe aminoalkyle, un groupe thioalkyle ou un groupe benzyle et n = 1 ou 2,
et
Y représente un atome d'oxygène,
ainsi que leurs hydrates et leurs sels d'addition d'acides pharmaceutiquement acceptables, ainsi que les sels de métaux alcalins, de métaux alcalino-terreux, d'argent et de guanidium des acides carboxyliques de base.

4. Composés répondant à la formule (I), selon la revendication 1, dans lesquels
R¹ représente un atome d'hydrogène, un groupe alkyle en C₁-C₄ ou un groupe (5-méthyl-2-oxo-1,3-dioxol-4-yl)-méthyle,
R² représente un atome d'hydrogène, un groupe méthyle, un groupe (5-méthyl-2-oxo-1,3-dioxol-4-yl)-méthyle, des radicaux répondant aux structures -CH=CH-COOR³, -CH₂CH₂COOR³, -CH₂CH₂CN, -CH₂COCH₃, dans lesquelles R³ représente un groupe méthyle ou un groupe éthyle, ou encore un radical de structure générale R⁴-(NH-CHR⁵-CO)ₙ dans laquelle R⁴ représente un atome d'hydrogène, un groupe alkyle en C₁-C₃, ou encore le radical -COO- tert.-butyle, R⁵ représente un atome d'hydrogène, un groupe alkyle en C₁-C₄, un groupe hydroxyalkyle, un groupe aminoalkyle, un groupe thioalkyle ou un groupe benzyle et n = 1 ou 2,
et
Y représente un atome d'oxygène,
ainsi que leurs hydrates et leurs sels d'addition d'acides pharmaceutiquement acceptables, de même que les sels de métaux alcalins, de métaux alcalino-terreux, d'argent et de guanidium des acides carboxyliques de base.

5. Acide 8-cyano-1-cyclopropyl-7-(2,8-diazabicyclo-[4.3.0]-nonan-8-yl)-6-fluoro-1,4-dihydro-4-oxo-3-quinoléinecarboxylique et ses esters tels qu'ils sont représentés dans les revendications 1, 3 et 4 pour les composés répondant à la formule (I).

6. Médicaments contenant des composés répondant à la formule (I) selon la revendication 1.

7. Utilisation de composés répondant à la formule (I) selon la revendication 1 pour la préparation de médicaments.

8. Utilisation selon la revendication 7, pour la préparation de médicaments destinés à lutter contre des bactéries Gram négatif et Gram positif et contre des micro-organismes analogues à des bactéries.
